# EUROPEAN PATENT APPLICATION

(11) **EP 1 405 612 A1**
(43) Date of publication of application: **07.04.2004**
(21) Application number: 02425596.0
(22) Date of filing: 02.10.2002
(51) Int. Cl.: A61F 2/04, A61F 5/00, A61B 17/11

(54) **Transanal device**

(71) Applicant: Sapi Med S.p.A., 15100 Alessandria (IT)
(72) Inventor: Montemurro, Severino, 70125 Bari (IT)
(74) Representative: Long, Giorgio

(57) **Abstract**

Transanal device (100) comprising a tubular element (1; 10) which rises in an essentially orthogonal way from a base element (2; 20), in which the said transanal device (100) is made from biocompatible elastic material and in which the said tubular element (1; 10) is provided with at least two longitudinal portions having different thicknesses from each other.

## Description

The present invention relates to a transanal device to be used for lining an area of the anal canal.

In particular, the present invention relates to a transanal device to be used for lining an area of the anal canal subjected to anastomosis, and more particularly to coloanal or colorectal anastomosis.

It is known that a colorectal or coloanal anastomosis is generally performed following partial or total resection of the sigmoid colon and/or rectum for removing neoplastic pathologies located therein.

It is also known that these anastomoses are associated with a wide range of complications, the most dangerous of which, because of its clinical consequences, is fistula, which has proved to be the cause of higher postoperative mortality in cases of removal of rectal cancer.

This is because the peristaltic motion, on the one hand, and the sphincter tone, on the other hand, subject the area of the anastomosis to trauma which tends to weaken the said area.

This is aggravated by the fact that, in the initial postoperative period, the absence of the anal inhibitory reflex causes the faeces being passed to impact against a closed sphincter. Thus the area of the anastomosis is subjected to additional trauma due to the progressive accumulation of gas and faeces.

Consequently, fistulas formed in this way in the area of the anastomosis cause an escape of gas and faeces into the abdomen, which may result in the death of the affected person.

Up to the present time, colorectal or coloanal anastomoses have been performed in association with a diversion of the passage of faeces to the exterior of the abdomen in order to overcome the aforesaid problem, or in other words to prevent the area of the anastomosis from being subjected to the aforesaid trauma. Typical examples of such diversions are ileostomy and colostomy.

However, such stomata have numerous disadvantages, including the exposure of the patient to disorders associated with the stoma itself, such as laparocele, stenosis and skin irritation, problems with the management of base plates and pouches for the stoma, associated in turn with specific disorders such as stenosis, fistula, laparocele and cutaneous suppurations, and, last but not least, psychological and social problems in the patient.

The problem upon which the present invention is based is that of overcoming the aforesaid disadvantages.

This problem is resolved by a transanal device as described in the attached claims.

Further characteristics and advantages of the transanal device according to the present invention are made clearer by the following description of the preferred embodiments, given by way of non limiting example, with reference to the following figures:
Figure 1 is a sectional view of the transanal device according to the present invention, in a first embodiment;
Figure 2 is a sectional view of the transanal device of Figure 1 in use;
Figure 3 is a sectional view of the transanal device according to the present invention, in a second embodiment;
Figure 4 is a sectional view of the transanal device of Figure 3 in use.

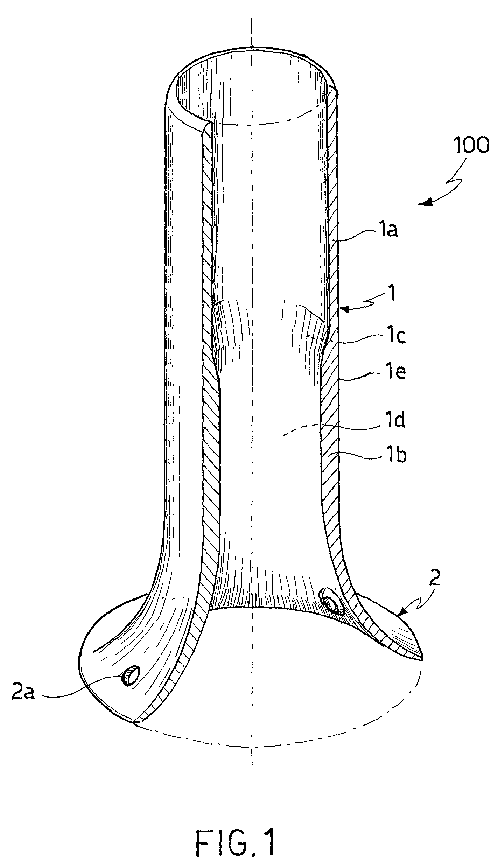

As shown in Figures 1, 2, 3 and 4, the transanal device according to the present invention, indicated as a whole by the number 100, is made from biocompatible elastic material.

A preferred example of the said elastic material is a silicone polymer.

The said transanal device 100 according to the present invention comprises a tubular element 1; 10 which rises in an essentially orthogonal way from a base element 2; 20, and has at least two different thicknesses along the longitudinal portion of the said tubular element 1; 10, in such a way as to line the wall of the anal canal in a non-traumatic way while also permitting defecation.

In the present description and in the claims, the expressions "in a non-traumatic way" and "non-traumatic" mean that the tubular element 1; 10 adheres fully to the wall of the anal canal which contains it without subjecting this wall to tensile stress.

Preferably, the said transanal device 100 has at least three different thicknesses along the longitudinal portion of the said tubular element 1; 10.

Advantageously, the said tubular element 1; 10 is thinner in the area of the anal canal subjected to anastomosis. This is because this ensures that the aforesaid area is lined with a portion of the tubular element which is flexible and elastic and is therefore soft.

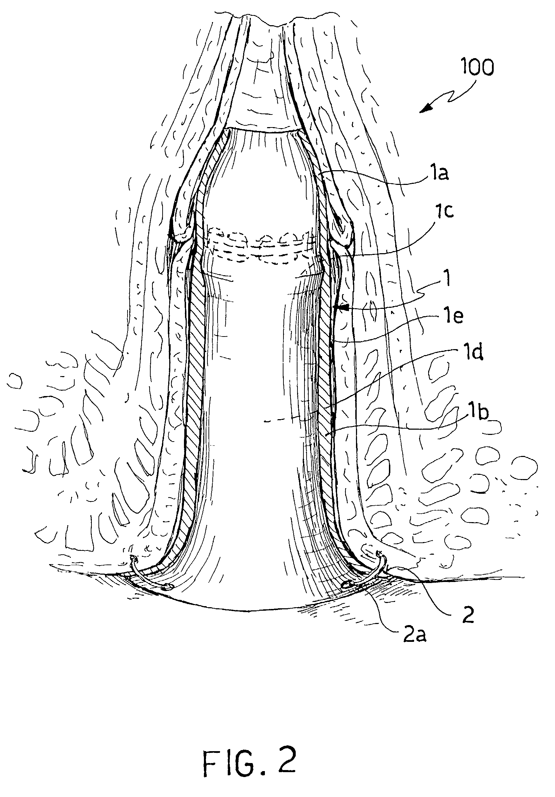

In a first embodiment (Figures 1 and 2), the said tubular element 1 of the transanal device 100 is formed by at least one lower tubular portion 1b, joined to the base element 2 and at least one upper tubular portion 1a, the said at least one lower tubular portion 1b and upper portion 1a having constant internal diameters which are different from each other.

The said at least one lower tubular portion 1b has a constant internal diameter which is smaller than that of the said at least one upper tubular portion 1a.

As shown in Figures 1 and 2, the said at least one upper tubular portion 1a and the said at least one lower tubular portion 1b are joined together by at least one inclined tubular portion 1c of truncated conical shape.

The said at least one inclined tubular portion 1c has a diameter of the larger base of the truncated cone equal to the diameter of the upper tubular portion 1a, and a diameter of the smaller base of the truncated cone equal to the diameter of the lower tubular portion 1b.

The said tubular element 1 has an inner wall 1d and an outer wall 1e.

The said inner wall 1d has a uniformly smooth surface, while the said outer wall 1e has a slightly rough surface.

Advantageously, the said tubular element 1 is thinner in the said at least one upper tubular portion 1a and thicker in the said at least one lower tubular portion 1b. Therefore, as stated above, the said upper tubular portion 1a is more flexible than the lower tubular portion 1b, as shown in Figure 2.

In particular, the said at least one lower tubular portion 1b has a thickness of at least 1.5 mm and the said at least one upper tubular portion 1a has a thickness of at least 0.5 mm.

More particularly, the said at least one lower tubular portion 1b has a thickness in the range from 1.5 to 2.5 mm and the said at least one upper tubular portion 1a has a thickness in the range from 0.5 to 1.5 mm.

In one embodiment of the said Figures 1 and 2, the said tubular element 1 has a length of 80 mm, with a lower tubular portion 1b measuring approximately 42.5 mm, an upper tubular portion 1a measuring approximately 34.5 mm, and an inclined tubular portion 1c measuring approximately 3 mm; the upper tubular portion 1a has an internal diameter of approximately 15.5 mm, and the lower tubular portion 1b has an internal diameter of approximately 14.5 mm; the wall of the lower tubular portion 1b has a thickness of 2 mm, and the wall of the upper tubular portion 1a has a thickness of 1 mm.

The base element 2 is of circular shape and protrudes outside the tubular element 1.

The said base element 2 has a constant thickness which is in the range from 0.5 to 1.5 mm, and is preferably 1 mm.

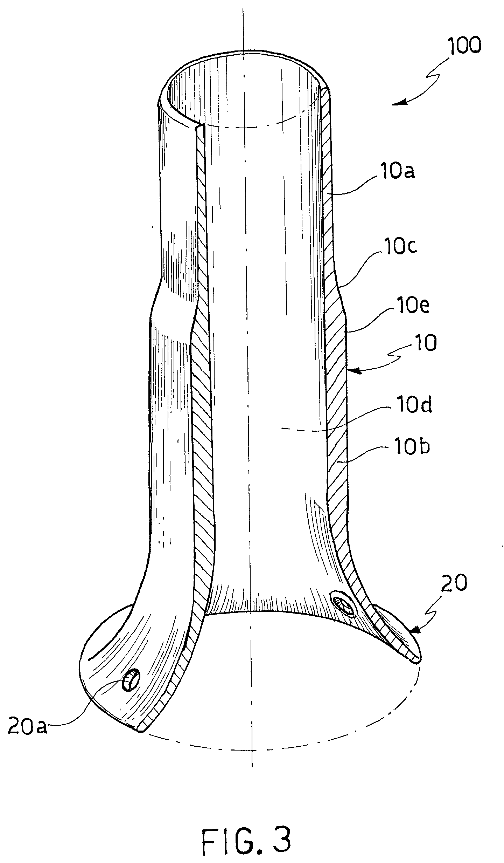

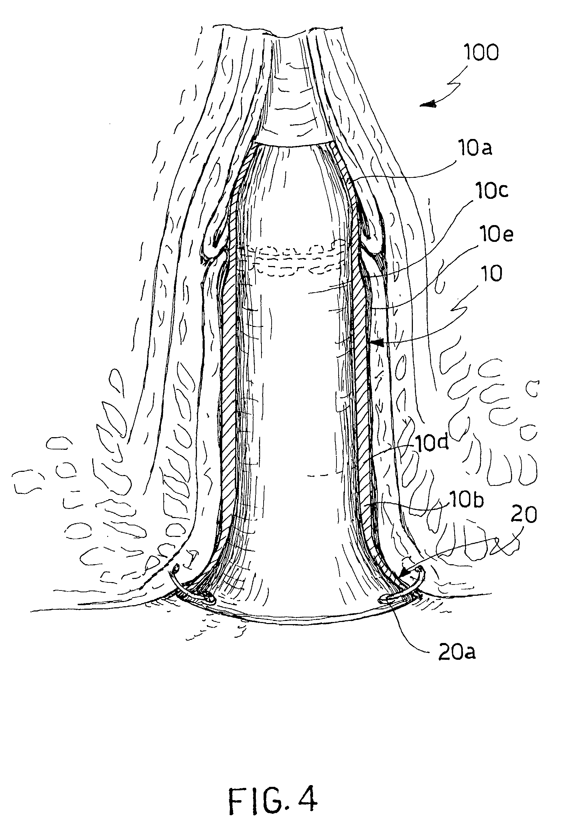

In a second preferred embodiment (Figures 3 and 4), the said tubular element 10 of the transanal device 100 is formed by at least one lower tubular portion 10b, joined to the base element 20 and at least one upper tubular portion 10a.

Advantageously, the said tubular element 10 is thinner in the said at least one upper tubular portion 10a and thicker in the said at least one lower tubular portion 10b. Therefore, as stated above, the said upper tubular portion 10a is more flexible than the lower tubular portion 10b, as shown in Figure 4.

Advantageously, as shown in Figures 3 and 4, the said at least one upper tubular portion 10a and the said at least one lower tubular portion 10b of the tubular element 10 have internal diameters which are constant and equal to each other. Thus the gas and faeces passing through the inside of the tubular element 10 when in use do not encounter any obstacles.

The said tubular element 10 also has an inner wall 10d and an outer wall 10e.

The said inner wall 10d has a uniformly smooth surface, free of any projections and/or recesses, while the said outer wall 10e has a slightly rough surface.

Additionally, as shown in Figures 3 and 4, the said outer wall 10e has at least one inclined tubular portion 10c which joins the said at least one upper tubular portion 10a to the said at least one lower tubular portion 10b.

In particular, the said at least one lower tubular portion 10b has a thickness of at least 1.5 mm and the said at least one upper tubular portion 10a has a thickness of at least 0.5 mm.

More particularly, the said at least one lower tubular portion 10b has a thickness in the range from 1.5 to 2.5 mm and the said at least one upper tubular portion 10a has a thickness in the range from 0.5 to 1.5 mm.

In one embodiment of the said Figures 3 and 4, the said tubular element 10 has a length of 60 mm, with a lower tubular portion 10b approximately 25 mm in length, an upper tubular portion 10a approximately 18 mm in length, and an inclined tubular portion 10c approximately 5 mm in length with a constant internal diameter of approximately 16.5 mm; the lower tubular portion 10b has a thickness of 2 mm; and the upper tubular portion 10a has a thickness of 1 mm.

The base element 20 is of circular shape and protrudes outside the tubular element 10.

The said base element 20 has a constant thickness which is in the range from 0.5 to 1.5 mm, and is preferably 1 mm.

The said transanal device 100 according to the present invention is therefore useful for lining an area of the canal, since it can cover this area in a non-traumatic way while also permitting defecation.

In use, the transanal device 100 according to the present invention is applied to the anal canal of a patient by inserting the tubular element 1; 10 into the anal canal in such a way that the whole base element 2; 20 is positioned outside the said canal and in contact with the skin of the perineum.

Preferably, the said base element 2; 20 is fixed to the skin of the perineum by suturing. Even more preferably, the said base element 2; 20 is fixed to the skin of the perineum by means of two sutures, one on the right-hand side and one on the left-hand side.

Advantageously, the said base element 2; 20 can be provided with two eyelets 2a; 20a located at 180° to each other at the position of the two aforesaid sutures, to prevent the sutures from being pulled out.

The transanal device 100 according to the present invention is inserted into the patient while the latter is under general anaesthesia, and is kept in the anal canal for the whole of the period required for the area of the anastomosis to become stabilized. It is generally removed on the sixth day after the operation.

The selected shape and dimensions of the transanal device 100 according to the present invention are the result of extensive testing conducted by the applicant.

Unexpectedly, the applicant has found that the aforesaid shapes and dimensions enable an area of the anal canal to be lined in a non-traumatic way, because the transanal device according to the present invention is capable of reducing the pressure within the anal canal.

In particular, the applicant has observed that, when the transanal device 100 according to the present invention is in place, the pressure within the anal canal is reduced to values of 1-2 mmHg measured by anorectal manometry with a low compliance perfusion pump.

The transanal device 100 according to the present invention can therefore reduce both the pressure normally exerted by peristaltic motion, which is generally at least 80-100 mmHg, and that due to sphincter tone, which is generally approximately 10 mmHg.

Thus the percentage of patients with fistula formation following anastomosis is drastically reduced.

The advantages of the transanal device according to the present invention are immediately clear from the above description.

A first advantage is that the presence of a plurality of portions of the tubular element having different thicknesses in the transanal device according to the present invention decreases the pressure within the anal canal, thus reducing the percentage of fistula formation in the area of the anastomosis while not impeding defecation.

A second advantage is that the internal diameter and the thickness of the upper tubular portion of the transanal device according to the present invention enable this upper tubular portion to be positioned in such a way that it adheres fully and in a non-traumatic way to the area subjected to anastomosis. Thus the formation of decubitus both in the area of the anastomosis itself and in the surrounding area is avoided.

A third advantage is that the shape of the inclined tubular portion with a tapered wall enables the lumen to be kept open, in opposition to the contracting force of the sphincters.

A fourth advantage is that the presence of portions of the tubular element with different thicknesses causes the element to adhere fully to the inner wall of the anal canal. This helps to avoid subjecting the anastomosis to tensile forces which might cause the formation of fistulas.

A fifth advantage is that the inner wall of the tubular element is smooth. This facilitates the sliding within the element of material to be expelled, such as faeces, thus avoiding the accumulation of deposits in the element which might cause the blocking of the said transanal device.

A further advantage is that the outer wall of the tubular element is slightly rough. This provides a stable contact of the said device with the inner wall of the anal canal. This prevents the material sliding through the said tubular element from becoming trapped between the said outer wall and the inner wall of the anal canal, which would cause infection and irritation of the canal.

Another advantage is that the shape and thickness of the base element permits a full adhesion of this element to the skin of the perineum. Thus the formation of decubitus on the skin is avoided.

Because there are at least two longitudinal portions having different thicknesses from each other in the tubular element of the device according to the present invention, it is possible to provide a device capable of counterbalancing the different peristaltic pressures present along the wall of the said canal. Thus the tubular element can support the aforesaid wall of the anal canal in a uniform way. This is achieved by providing, in the area of the anal canal subjected to anastomosis, a portion of the tubular element which is thinner and therefore has greater flexibility than the other portions of the said tubular element, while, on the other hand, providing, in the area of the anal canal closer to the anal sphincter, a portion of the tubular element which is thicker and therefore stronger than the other portions of the tubular element according to the present invention. Thus the natural tendency of the wall of the anal canal to close up is opposed in a non-traumatic way, since the portions of the tubular element reinforce the wall of the said canal in different ways, according to both the anatomy and the pathology of the said anal canal, while also permitting defecation.

Clearly, the embodiments described above are only some particular embodiments of the transanal device according to the present invention, and a person skilled in the art will be able to make any necessary modifications to the invention in order to adapt it to particular applications, without departure from the scope of protection of the present invention.

## Claims

1. Transanal device (100) comprising a tubular element (1; 10) which rises in an essentially orthogonal way from a base element (2; 20), in which the said transanal device (100) is made from biocompatible elastic material and in which the said tubular element (1; 10) is provided with at least two longitudinal portions having different thicknesses from each other.

2. Transanal device (100) according to Claim 1, in which the said at least two longitudinal portions can line the wall of the anal canal in a non-traumatic way while also permitting defecation.

3. Transanal device (100) according to Claim 1 or 2, in which the said biocompatible elastic material is a silicone polymer.

4. Transanal device (100) according to any one of Claims 1 to 3, in which the said tubular element (1; 10) fully lines the wall of the anal canal which contains it.

5. Transanal device (100) according to any one of Claims 1 to 4, in which the said tubular element (1; 10) has at least three different thicknesses along its longitudinal portion.

6. Transanal device (100) according to any one of Claims 1 to 5, in which the said tubular element (1; 10) is thinner in the area of the anal canal subjected to anastomosis.

7. Transanal device (100) according to any one of Claims 1 to 6, in which the said tubular element (1; 10) is formed by at least one lower tubular portion (1b; 10b), joined to the said base element (2; 20), and by at least one upper tubular portion (1a; 10a).

8. Transanal device (100) according to Claim 7, in which the said at least one lower tubular portion (1b) and the said at least one upper tubular portion (1a) have constant diameters which are equal to each other.

9. Transanal device (100) according to any one of Claims 1 to 8, in which the said at least one lower tubular portion (1b; 10b) and the said at least one upper tubular portion (1a; 10a) are joined together by means of an inclined tubular portion (1c: 10c).

10. Transanal device (100) according to Claim 9, in which the thickness of the wall of the said lower tubular portion (1b; 10b) is at least 1.5 mm.

11. Transanal device (100) according to Claim 10, in which the thickness of the wall of the said lower tubular portion (1b; 10b) is in the range from 1.5 to 2.5 mm.

12. Transanal device (100) according to Claim 9, in which the thickness of the wall of the said upper tubular portion (1a; 10a) is at least 0.5 mm.

13. Transanal device (100) according to Claim 12, in which the thickness of the wall of the said upper tubular portion (1a) is in the range from 0.5 to 1.5 mm.

14. Transanal device (100) according to any one of Claims 1 to 13, in which the said tubular element (1; 10) is provided with an outer wall (1e; 10e) having a slightly rough surface.

15. Transanal device (100) according to any one of Claims 1 to 14, in which the said tubular element (1; 10) is provided with an inner wall (1d; 10d) having a surface which is uniformly smooth and free of projections and/or recesses.

16. Transanal device (100) according to any one of Claims 1 to 15, in which the said base element (2; 20) is of circular shape and protrudes outside the said tubular element (1; 10).

17. Transanal device (100) according to Claim 16, in which the said base element (2; 20) has a constant thickness in the range from 0.5 to 1.5 mm.

18. Use of the transanal device (100) described in Claims 1 to 17 for lining an area of the anal canal.

19. Use according to Claim 18, in which the area of the anal canal is subjected to anastomosis.

20. Use according to Claim 19, in which the anastomosis is of the colorectal or coloanal type.
